# EUROPÄISCHE PATENTSCHRIFT

(11) **EP 2 842 504 B1**
(45) Veröffentlichungstag und Bekanntmachung des Hinweises auf die Patenterteilung: **11.07.2018**
(21) Anmeldenummer: 14182914.3
(22) Anmeldetag: 29.08.2014
(51) Int. Cl.: A61B 17/86, A61B 17/80

(54) **Knochenschraube, Implantatplatte und System daraus**
Bone screw, implant plate and a system comprising same
Vis à os, plaque implantable et système formé des deux

(30) Priorität: 02.09.2013 DE 102013109569
(43) Veröffentlichungstag der Anmeldung: 04.03.2015
(73) Patentinhaber: AESCULAP AG, 78532 Tuttlingen (DE)
(72) Erfinder: Saueressig, Thomas, 78532 Tuttlingen (DE)
(74) Vertreter: Winter, Brandl, Fürniss, Hübner, Röss, Kaiser, Polte - Partnerschaft mbB

(56) Entgegenhaltungen:
- EP-A1- 2 364 657
- US-A1- 2007 233 125
- US-A1- 2009 157 123
- US-A1- 2011 184 415
- US-A1- 2011 313 421

## Beschreibung

Die vorliegende Erfindung betrifft eine Knochenschraube zum winkelvariablen bzw. polyaxialen Verschrauben einer Implantatplatte mit einem Knochen. Sie betrifft ferner eine Implantatplatte für eine derartige Verschraubung sowie schließlich ein System aus einer solchen Knochenschraube und einer solchen Implantatplatte.

Polyaxiale bzw. multidirektionale Verschraubungen einer Knochenschraube in einer Knochen- oder Implantatplatte sowie Knochenschrauben und Implantatplatten dafür sind aus dem Stand der Technik bekannt. Bei bekannten Verschraubungen besteht oftmals der Nachteil von Spanbildung beim Eindrehen der Schraube, weil die Gewinde von Knochenschraube und -platte insbesondere bei einem schrägen Eindrehen nicht richtig zusammenpassen und sich das Gewinde der Schraube in das Material der Knochenplatte schneidet. Ein weiterer Nachteil kann sein, dass die erzielte Verbindung instabil werden kann, da der Schraubenkopf in einem zu geringen Eingriff mit der Platte steht und deswegen relativ zu dieser unzureichend gesichert ist. Dies kann zur Folge haben, dass die Schrauben nach einer gewissen Zeit lateral aus der Platte heraus in angrenzende Weichteile wandern, wenn ihr Halt im Knochen, in den sie eingeschraubt sind, nachlässt.

Die EP2 308 403 B1 offenbart eine Knochenschraube mit einem ein Knochengewinde aufweisenden Schaft und mit einem axial benachbart zum Knochengewinde angeordneten Kopfgewinde mit konischer Hüllstruktur. Das Kopfgewinde läuft an einem vom Knochengewinde abgewandten Ende in einen umlaufenden Freistich aus. Der Freistich weist eine Freistichflanke auf, die für ein Verblocken mit der Knochenplatte bestimmt ist. Dabei kann Kaltverschweißen verhindert werden, da die Verblockung unmittelbar nach Auftreffen der Freistichflanke auf die Knochenplatte erfolgt. Bei dieser Verschraubung besteht der Nachteil, dass der Schraubenkopf nur über einen kleinen Bereich mit der Knochenplatte in Eingriff steht. Bei einem schrägen Einschrauben der Knochenschraube in die Knochenplatte kann es trotz des Freistichs zur Spanbildung kommen, da das Kopfgewinde der Knochenschraube beim Eindrehen in das Material der Knochenplatte einschneidet und es zu Materialabtragung kommt.

Aus der DE 10 2005 042 766 A1 ist eine winkelstabil variable Verschraubung für eine Knochenplatte zur Osteosynthese bekannt. In der Knochenplatte ist eine sternförmige Öffnung mit einem Innengewinde ausgebildet. Dieses besteht aufgrund der Sternform aus Gewindeabschnitten und jeweils zwischen zwei benachbarten Gewindeabschnitten ausgebildeten Ausbuchtungen. Der Gewindekerndurchmesser verjüngt sich in Einschraubrichtung. An der Oberseite der Knochenplatte befindet sich eine gewindefreie Pfanne zur Aufnahme eines Schraubenkopfs. Die Knochenplatte kann mit einer Rund- oder mit einer Gewindekopfschraube genutzt werden, wobei letztere winkelstabil in der Plattenöffnung arretierbar ist, die Rundkopfschraube jedoch nicht. Wird eine Gewindekopfschraube schräg in die Plattenöffnung eingeschraubt, schneidet das Kopfgewinde der Schraube teilweise in die Gewindeabschnitte der sternförmigen Öffnung ein, wodurch es mit Nachteil zur Materialabtragung und zur Spanbildung kommt.

Aus der US 2006/0009771 A1 ist ein Stabilisierungssystem für Knochen bekannt. Dieses weist eine im Wesentlichen T-förmige Knochenplatte mit mit einem Innengewinde versehenen Durchgangsöffnungen sowie einen Satz vorbestimmt unidirektional eindrehbarer Schrauben und einen Satz durch einen Chirurgen wählbar multidirektional eindrehbarer Schrauben auf. Die Schrauben weisen in verschiedenen Ausführungsformen mit einem Kopfgewinde versehene Schraubenköpfe auf. Bei einem schrägen Einschrauben in die Durchgangsöffnungen der Platte schneidet das Kopfgewinde in das Material der Platte, insbesondere in das Innengewinde in der jeweiligen Durchgangsöffnung, wodurch es mit Nachteil zur Spanbildung kommt.

Aus der WO2011/085272 A1 ist ein winkelvariables Knochenschraubensystem bekannt. Das System besteht aus einer Knochenplatte mit einer darin eingebrachten Durchgangsöffnung, einer Knochenschraube mit einem Knochen- und einem Kopfgewinde sowie einer napfförmigen Distanzscheibe mit Innengewinde. Die Durchgangsöffnung ist mit einem teilkugelförmigen Flansch versehen, dessen Kontur passend zu der der Distanzscheibe ist. Diese muss durch den Chirurgen von der Unterseite der Knochenplatte in die Durchgangsöffnung eingebracht werden, woraufhin ihre napfförmige Kontur an dem teilkugelförmigen Flansch anliegt. Die Knochenschraube wird von der gegenüberliegenden Seite in die Durchgangsöffnung eingebracht. Deren Knochengewinde ist kleiner als das Innengewinde der Distanzscheibe, so dass zwischen diesen kein Eingriff erfolgt. Das Kopfgewinde der Knochenschraube jedoch greift in das Innengewinde der Distanzscheibe ein. Bei einem tiefen Eindrehen der Knochenschraube wird, wenn der Kopf der Knochenschraube gegen die Oberseite der Knochenplatte stößt, durch diesen Eingriff die Distanzscheibe gegen den teilkugelförmigen Flansch der Knochenplatte gepresst. Die Distanzscheibe und damit auch die darin eingeschraubte Knochenschraube sind dann gegenüber der Knochenplatte lagefixiert. Aufgrund der Teilkugelflächen von Distanzscheibe und Flansch ist das System winkelvariabel und unabhängig vom Einschraubwinkel fixierbar. Da die das Innengewinde der Distanzscheibe und das Kopfgewinde der Knochenschraube immer passend zueinander ausgerichtet sind, kommt es nicht zur Spanbildung. Allerdings weist das System zahlreiche Komponenten auf, ist aufwendig herzustellen und hinsichtlich seiner Handhabung komplex.

In der US 2007/0233125 A1 ist eine Knochenschraube mit einem separaten Sicherungskopf offenbart, mittels welcher zwei Knochenteile relativ zueinander ohne Zuhilfenahme einer Implantatplatte fixiert werden können.

In der US 2009/0157123 A1 wird ein System bestehend aus einer Implantatplatte und Knochenschrauben offenbart. Die Knochenschrauben gemäß US 2009/0157123 A1 sind derart flexibel ausgebildet, dass die zu verbindenden Knochenteile oder -fragmente während des Fixierens nicht durch allzu große Spannungen beschädigt werden.

In den Druckschriften US 2011/0184415 A1 und US 2011/0313421 A1 sind Implantatplatten mit Durchgangslöchern offenbart, wobei an den Durchgangslöchern Krägen vorgesehen sind, mittels derer Knochenschrauben eingeklemmt werden können.

Ausgehend vom vorstehend beschriebenen Stand der Technik liegt der Erfindung die Aufgabe zugrunde, einem Chirurgen zu ermöglichen, Knochenschrauben je nach Frakturverlauf in gewünschter Richtung, d.h. variabel multidirektional, in eine Implantatplatte und einen Knochen einzuschrauben. Dabei soll Spanbildung nicht auftreten und die Knochenschraube sicher und dauerhaft gegenüber der Implantatplatte gesichert oder verblockt sein, so dass ein postoperatives Lösen der Schraube und damit deren laterales Auswandern in angrenzende Weichteile verhindert oder zumindest verlangsamt werden. Die Verbindung soll auch nach einer gewissen Zeit, z.B. im Rahmen einer Implantatentfernung, einfach wieder zu lösen sein. Das System und seine Komponenten sollen einfach und zu günstigen Kosten herzustellen und insbesondere für den sie nutzenden Chirurgen einfach anzuwenden sein.

Die Aufgabe wird gelöst durch eine Knochenschraube gemäß den Ansprüchen 1 und 2, durch eine Implantatplatte gemäß dem Anspruch 7 und ein System gemäß dem Anspruch 14. Die Aufgabe wird gelöst durch eine Knochenschraube zum winkelvariablen Verschrauben einer Implantatplatte mit einem Knochen, wobei die Knochenschraube einen Schaft mit einem Knochengewinde und an einem Ende des Schafts einen Schraubenkopf aufweist, wobei in dem Schraubenkopf an dessen zum Schaft weisenden Seite eine in Umfangsrichtung umlaufende nutförmige Vertiefung ausgebildet ist, welche nutförmige Vertiefung eine Nutflanke aufweist, die zum Verklemmen, Verspannen oder Verblocken mit einer Implantatplatte bestimmt ist. Die Knochenschraube ist insbesondere geeignet und bestimmt zum winkelvariablen Verschrauben mit einer Implantatplatte nach einem der Ansprüche 7 bis 13.

Die Aufgabe wird des Weiteren gelöst durch eine Implantatplatte zum winkelvariablen Verschrauben einer Knochenschraube mit einem Knochen, wobei die Implantatplatte wenigstens eine Durchgangsöffnung zur Aufnahme der Knochenschraube aufweist, wobei die Durchgangsöffnung auf der der zur Anlage am Knochen bestimmten Seite gegenüberliegenden Seite der Implantatplatte von einem in Umfangsrichtung umlaufenden und in axialer Richtung vorstehenden Kragen umgeben ist, wobei der Kragen eine Kragenflanke aufweist, die zum Verklemmen, Verspannen oder Verblocken mit der Knochenschraube bestimmt ist. Die Implantatplatte ist insbesondere zum Verschrauben mit einer Knochenschraube nach einem der Ansprüche 1 bis 6 bestimmt.

Die nutförmige Vertiefung der Knochenschraube kann als Hinterschneidung ausgebildet sein. Nach der Erfindung greift bei bestimmungsgemäßer Verwendung der Knochenschraube zumindest ein Abschnitt oder eine entsprechende Gegenstruktur der Implantatplatte in diese Hinterschneidung ein. Die Formen der Hinterschneidung und des wenigstens einen Abschnitts der Implantatplatte sind derart ausgebildet, dass beim Eindrehen der Knochenschraube die Hinterschneidung und der genannte Abschnitt bzw. die genannten Abschnitte der Implantatplatte in Einschraubrichtung gegeneinander bewegt werden und aufgrund ihrer aufeinander abgestimmten Geometrie miteinander Verklemmen, Verspannen oder Verblocken. Es ist von besonderem Vorteil, dass dieses ohne Bildung von Spänen, die bei Verschraubungen nach dem Stand der Technik von der Schraube oder der Implantatplatte z.B. durch Einschneiden des Schrauben- oder Implantatgewindes in das Material des jeweiligen Gegenstücks gelöst werden, ablaufen kann. Mit besonderem Vorteil erfolgt das Verklemmen, Verspannen oder Verblocken automatisch beim Eindrehen der Knochenschraube, ohne dass neben der Knochenschraube weitere Elemente, z.B. in Form von Muttern, Klemmelementen oder ähnlichem, durch den Operateur gehandhabt werden müssen. Das System nach der Erfindung ist daher besonders einfach und sicher zu handhaben. Gemäß der Erfindung ist die zum Verklemmen, Verspannen oder Verblocken bestimmte Nutflanke der Vertiefung die in radialer Richtung äußere Nutflanke. Dadurch wird ermöglicht, dass die Knochenschraube auch bei einem schrägen Eindrehen unabhängig vom jeweiligen Eindrehwinkel in die Implantatplatte sicher mit deren dazu bestimmten Struktur verklemmt, dass die Schraube in unterschiedlichen, vom Chirurgen individuell wählbaren Winkeln relativ zur Implantatplatte eingeschraubt werden kann und es des Weiteren nicht zur Spanbildung kommt. Gemäß der Erfindung ist die zum Verklemmen, Verspannen oder Verblocken bestimmte Nutflanke in axialer Richtung gegenüber der Längsachse des Schaftes um einen Winkel α geneigt. Der Winkel α liegt in einem Bereich zwischen 30° und 50°, vorzugsweise zwischen 35° und 45°. Die Nutflanke kann gerade oder gewölbt ausgebildet sein. Um verschiedene Einschraubwinkel der Knochenschraube relativ zur Implantatplatte zu ermöglichen, wobei stets ein sicheres Verklemmen gewährleistet ist, ist nach einer Form der Erfindung die zum Verklemmen, Verspannen oder Verblocken bestimmte Nutflanke nicht nur in Umfangsrichtung gekrümmt, sondern (teil-)sphärisch bzw. konkav ausgebildet. Sie ist vorzugsweise kugelsegmentförmig mit einem Radius "r" ausgebildet. Der Mittelpunkt der Segmentkrümmung liegt auf der Mittelachse des Schraubenschaftes, d.h. der Radius "r" verläuft von der Schaftachse zur Oberfläche der gekrümmten Nutflanke. Der Radius "r" ist vorzugsweise derart ausgewählt, dass der Mittelpunkt der Segmentkrümmung den bestimmungsgemäßen Drehpunkt der Knochenschraube ausbildet, um den diese bei einem schrägen Einschrauben gegenüber einer Normalen der in der Implantatplatte vorgesehenen Öffnung geschwenkt ist. Der Radius kann vorzugsweise so gewählt sein, dass sich der Drehpunkt der Schraube in einem proximalen Schaftabschnitt der Knochenschraube befindet, und zwar in demjenigen Schaftabschnitt, der sich in der Implantatplatte befindet, so dass sich der Dreh- oder Schwenkpunkt der Knochenschraube innerhalb der Implantatplatte befindet.

Um einen sicheren und festen Sitz der Knochenschraube in der Implantatplatte auch bei einem schrägen Einschrauben bewirken zu können, ist nach einer Form der Erfindung vorgesehen, dass sich das Knochengewinde möglichst über die gesamte Länge des Schaftes erstreckt. Es ist bevorzugt, wenn unterhalb des Schraubenkopfs möglichst kein gewindefreier Bereich vorliegt. Nach der Erfindung kann sich das Knochengewinde sogar in den Bereich der nutförmigen Vertiefung des Schraubenkopfs erstrecken. Bei einem einteilig mit dem Schaft ausgebildeten Schraubenkopf kann dies aus fertigungstechnischen Gründen problematisch sein. Nach einer Ausführungsform der Erfindung ist die Knochenschraube daher zweiteilig ausgebildet, und der Schraubenkopf oder ein Teil davon am kopfseitigen Ende des Schaftes angeordnet und befestigt. Vorzugsweise ist der Schraubenkopf zumindest teilweise durch einen am kopfseitigen Ende des Schaftes angeordneten, den Schaft radial umgebender (Schraubenkopf-)Ring ausgebildet. Das kopfseitige Ende des Schafts kann einen halbfertigen, mit dem Schaft einstückig ausgebildeten Schraubenkopfsockel aufweisen, der durch den nachträglich auf- oder anbringbaren Schraubenkopfring komplettiert wird. Die Knochenschraube kann nach dieser Form der Erfindung hergestellt werden, indem nach einem Ausbilden des Knochengewindes am Schaft der Ring, der Schraubenkopf oder wenigstens Teile davon auf dem mit dem Knochengewinde versehenen Schaft angeordnet und befestigt werden. Die Befestigung kann auf unterschiedliche Weise erfolgen, z.B. durch Formschluss (z.B. Crimpen), Kraftschluss (Pressen) oder Materialschluss (z.B. Schweißen, Löten, Kleben) oder eine Kombination daraus. Vorzugsweise sind der Ring oder der Schraubenkopf oder die Teile davon auf den Schaft aufgepresst. Nach einer Form der Erfindung ist die radial äußere Flanke der nutförmigen Vertiefung durch den auf dem Schaft angeordneten Ring oder Schraubenkopf oder Teil davon ausgebildet. Alternativ kann die ganze nutförmige Vertiefung darin ausgebildet sein. Durch das nachträgliche Aufbringen des Rings kann das Gewinde viel näher an den Schraubenkopf gefräst werden. Das Gewinde kann bis innerhalb des Rings reichen bzw. der Ring kann einen Endabschnitt des Gewinde umfassen oder umgeben. Ein solches bis nahe an den Schraubenkopf reichendes Gewinde ermöglicht die Verwendung der erfindungsgemäßen Knochenschraube auch mit dünnen Platten.

Nach einer Form der Erfindung kann der Kragen der Implantatplatte in Form einer Erhebung auf der Oberfläche der Implantatplatte ausgebildet sein.

Gemäß der Erfindung ist der Kragen ausgebildet, indem in die der zur Anlage am Knochen bestimmten Seite der Implantatplatte gegenüberliegende Seite eine die Durchgangsöffnung in einem bestimmten Abstand umgebende Vertiefung ausgebildet ist. Deren zur Durchgangsöffnung weisende Vertiefungsflanke bildet die zum Verklemmen oder Verblocken mit der Knochenschraube bestimmte Kragenflanke aus. Im letztgenannten Fall kann die Knochenschraube mit Vorteil in die den Kragen umgebende Vertiefung versenkt werden, so dass sie nicht oder nur unwesentlich über die Implantatplatte übersteht. Der Kragen und die ihn umgebene Vertiefung kann so tief in die Implantatplatte eingebracht sein, dass der Kopf der Knochenschraube vollständig versenkbar ist. Gemäß der Erfindung ist die zum Verklemmen oder Verblocken bestimmte Kragenflanke der Implantatplatte die auf der der Durchgangsöffnung abgewandten Seite liegenden Kragenflanke. Die Kragenflanke kann gerade oder gewölbt ausgebildet sein. Sie kann in axialer Richtung gegenüber der Längsachse der Durchgangsöffnung um einen Winkel geneigt sein. Dieser Winkel ist auf die Neigung und/oder Form der zum Verklemmen, Verspannen oder Verblocken vorgesehenen Nutflanke der Vertiefung der Knochenschraube angepasst. Nach einer Form der Erfindung liegt er vorzugsweise in einem Bereich zwischen 20° und 40°, bevorzugter zwischen 25° und 35°.Er ist insbesondere kleiner als der Neigungswinkel der entsprechenden Nutflanke der Knochenschraube, da auf diese Weise ein Einschneiden der die umlaufende Vertiefung der Knochenschraube umgebenden Kante in das Material der Implantatplatte vermieden wird. Des Weiteren kann, um verschiedene Einschraubwinkel der Knochenschraube relativ zur Implantatplatte unter sicherem Verklemmen zu ermöglichen, nach einer Form der Erfindung die zum Verklemmen, Verspannen oder Verblocken bestimmte Kragenflanke nicht nur in Umfangsrichtung gekrümmt, sondern (teil-)sphärisch ausgebildet sein. Sie ist vorzugsweise kugelsegmentförmig mit einem Radius "R" ausgebildet. Dieser kann kleiner oder gleich dem Radius "r" der Vertiefung der Knochenschraube sein. Der Mittelpunkt der Segmentkrümmung der Kragenflanke liegt vorzugsweise auf der Mittelachse der Durchgangsöffnung. Der Radius "R" kann derart ausgewählt sein, dass der Mittelpunkt der Segmentkrümmung den bestimmungsgemäßen Drehpunkt der Knochenschraube ausbildet, um den diese bei einem schrägen Einschrauben gegenüber einer Normalen der in der Implantatplatte vorgesehenen Öffnung geschwenkt ist. Er ist insbesondere auf die Stärke der Implantatplatte abgestimmt.

Nach einer Ausführungsform der Erfindung ist in der Durchgangsöffnung der Implantatplatte ein Innengewinde ausgebildet, insbesondere ein kegelförmiges Innengewinde mit einem bevorzugten Kegelwinkel von 16° oder 18°. Das Gewinde der Implantatplatte und das Knochengewinde der Knochenschraube haben vorzugsweise zueinander Spiel, insbesondere Winkelspiel. Auf diese Weise kann die Schraube in verschiedenen Winkel in die Durchgangsöffnung eingeschraubt werden, ohne dass es zur Spanbildung kommt, weil beispielsweise das Knochengewinde in das Innengewinde der Implantatplatte einschneidet oder umgekehrt.

Eine weitere Möglichkeit, eine schräge Verschraubung der Knochenschraube zu ermöglichen, besteht nach der Erfindung darin, dass die Achse der Durchgangsöffnung oder wenigstens einer Durchgangsöffnung gegenüber der Normalen der Implantatplatte um einen Winkel β geneigt ist, wobei der Winkel β vorzugsweise in einem Bereich zwischen 0° und 10°, bevorzugter zwischen 2° und 8,2° liegt. Die Neigung der Durchgangsöffnung kann zusätzlich oder alternativ zum vorgenannten Gewindespiel und/oder der Konizität des Innengewindes vorgesehen sein.

Nach der Erfindung kann jede Ausführungsform der Knochenschraube mit jeder Ausführungsform der Implantatplatte in einem System verwendet werden. Durch die Erfindung wird in vorteilhafter Weise ermöglicht, dass eine Schraube notwendigenfalls auch mehrfach eingeschraubt werden kann, da die Schraube nicht durch ein Einschneiden in das Material der Implantatplatte verblockt, sondern sich mit dieser ohne Spanbildung verklemmt, verspannt oder verblockt, ohne dass es zu Beschädigungen oder Veränderungen der Gewinde von Knochenschraube und Implantatplatte kommt.

Weitere Merkmale und Vorteile der vorliegenden Erfindung ergeben sich aus der folgenden beispielhaften Beschreibung einer besonders bevorzugten Ausführungsform der Erfindung anhand der Figuren. Dabei zeigt:
Fig. 1 eine Ausführungsform des Systems nach der Erfindung mit zwei Knochenschrauben, die in eine Implantatplatte eingeschraubt sind, in einer seitlichen Ansicht, einer Aufsicht und einer Schnittansicht;
Fig. 2 eine der Knochenschrauben mit Implantatplatte aus Figur 1 im Detail in einer Schnittansicht der Figur 1;
Fig. 3 die andere Knochenschraube mit Implantatplatte aus Figur 1 im Detail in einer Schnittansicht der Figur 1;
Fig. 4 eine Knochenschraube nach einer Ausführungsform in einer Seiten- und einer Schnittansicht;
Fig. 5 eine Knochenschraube nach einer anderen Ausführungsform in einer Seiten- und einer Schnittansicht;
Fig. 6 eine Implantatplatte nach der Erfindung in einer Schnittansicht; und
Fig. 7 die Implantatplatte der Figur 6 in einer perspektivischen Darstellung.

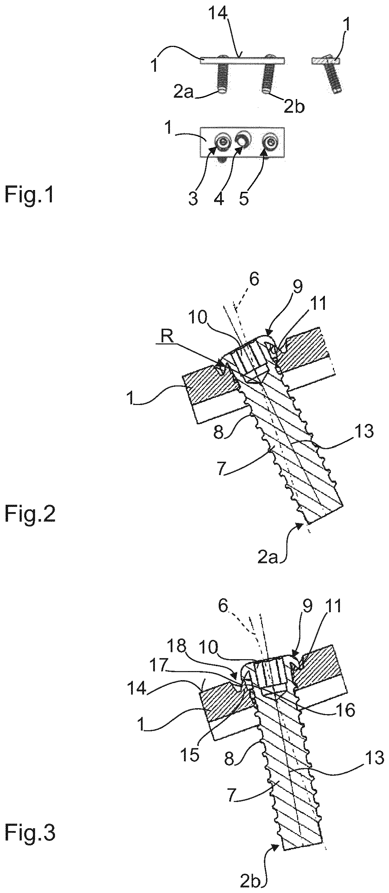

Die Figur 1 zeigt ein System nach der Erfindung, das eine Implantatplatte 1 und zwei Knochenschrauben 2a, b aufweist. Die Implantatplatte 1 weist drei Durchgangsöffnungen 3, 4, 5 auf, die zur Aufnahme einer Knochenschraube 2, 20 bestimmt sind. In die Durchgangsöffnungen 3 und 5 ist jeweils eine Knochenschraube 2a bzw. 2b eingebracht, während die Durchgangsöffnung 4 leer ist.

Die Knochenschraube 2a ist in Figur 2 zusammen mit der Implantatplatte 1 im Schnitt im Detail dargestellt. Die Knochenschraube 2b ist in Figur 3 zusammen mit der Implantatplatte 1 im Schnitt im Detail dargestellt. Aus einem Vergleich der Figuren 2 und 3 geht hervor, dass die Knochenschrauben 2a und 2b mit unterschiedlichen Einschraubwinkeln relativ zur Normalen 6 der Implantatplatte 1 positioniert sind.

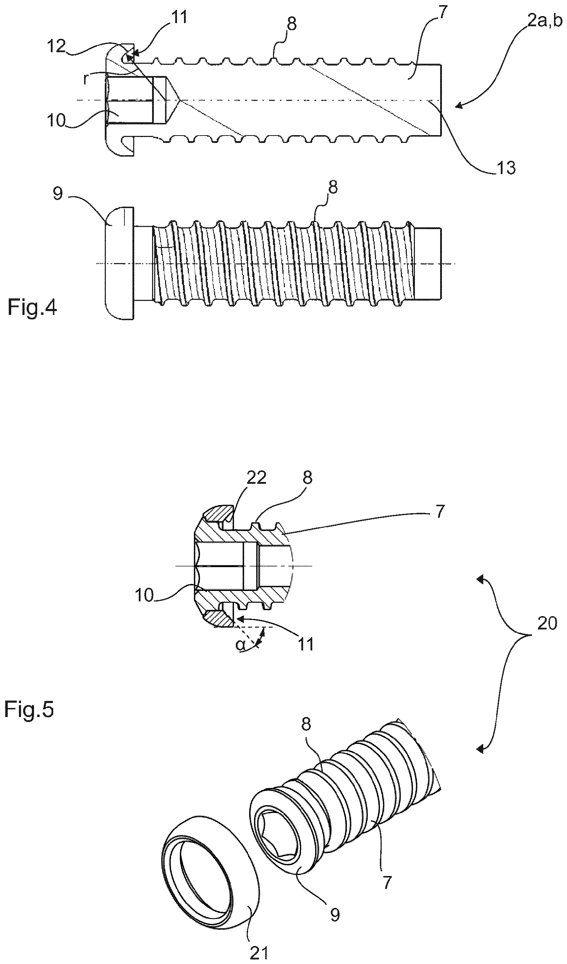

Die Knochenschraube 2a weist einen Schaft 7 auf (siehe auch Fig. 4), der mit einem Knochengewinde 8 versehen ist. Endseitig weist die Knochenschraube 2a einen Schraubenkopf 9 auf. Das Knochengewinde 8 läuft bis kurz unterhalb des Schraubenkopfs 9. In dessen vom Schaft 7 abgewandten Oberseite ist eine Aufnahme 10 für ein Schraubwerkzeug ausgebildet, im gezeigten Beispiel eine Innensechskantaufnahme für einen Sechskantschlüssel. In der dem Schaft 7 zugewandten Unterseite des Schraubenkopfs 9 ist eine nutförmige Vertiefung 11 ausgebildet. Diese läuft in Umfangsrichtung um, so dass sie eine ringförmige Nut bildet, die in der Unterseite des Schraubenkopfs 9 um den Schaft 7 herum verläuft. Die in radialer Richtung äußere Nutflanke 12 der Vertiefung 11 ist kugelsegmentartig, insbesondere konkav, geformt, wobei der Radius r des Kugelsegments von der Mittelachse 13 der Knochenschraube 2a bzw. des Schafts 7 zur Oberfläche der Nutflanke 12 verläuft. Die Knochenschraube 2b ist entsprechend ausgebildet.

An der Implantatplatte 1 ist an deren dem Knochen abgewandten Seite 14 um jede der Durchgangsöffnungen 3, 4, 5 ein die jeweilige Durchgangsöffnung ringförmig umgebender Kragen 15 ausgebildet. Der Kragen 15 weist eine der jeweiligen Durchgangsöffnung 3, 4, 5 zugewandte, also in radialer Richtung innere Kragenflanke 16 und eine der jeweiligen Durchgangsöffnung 3, 4, 5 abgewandte, also in radialer Richtung äußere Kragenflanke 17 auf. Die äußere Kragenflanke 17 ist kugelsegmentartig, insbesondere konvex, geformt, wobei der Radius R des Kugelsegments von der Achse 19 der Durchgangsöffnung 3, 4, 5 zur Oberfläche der Kragenflanke 17 verläuft. Genauer gesagt, befindet sich der Mittelpunkt des Kugelsegments in Dickenrichtung der Platte innerhalb der Implantatplatte, so dass der Schwenkpunkt der Knochenschraube 2a, b sich innerhalb der Implantatplatte befindet. Der Radiusmittelpunkt des Kugelsegments kann sich auch etwas außerhalb der Implantatplatte 1 befinden. Die beiden Radien r (der Nutflanke 12) und R (der Kragenflanke 17) können gleich groß sein, in welchem Fall - wie gezeigt - die beiden kugelsegmentartigen Flächen von Nutflanke 12 und Kragenflanke 17 vollflächig aneinander anliegen. Der Radius R der Kragenflanke 17 kann allerdings auch kleiner als der Radius r der Nutflanke 12 sein, in welchem Fall eine linienförmige Anlage von Nut- und Kragenflanke mit entsprechend größerer Flächenpressung erzielt wird und die Knochenschraube universeller verwendbar ist. Der Kragen 15 ist von einem ringförmigen Einstich 18 umgeben und wird durch Einbringen des Einstichs 18 in die Seite 14 der Implantatplatte 1 hergestellt.

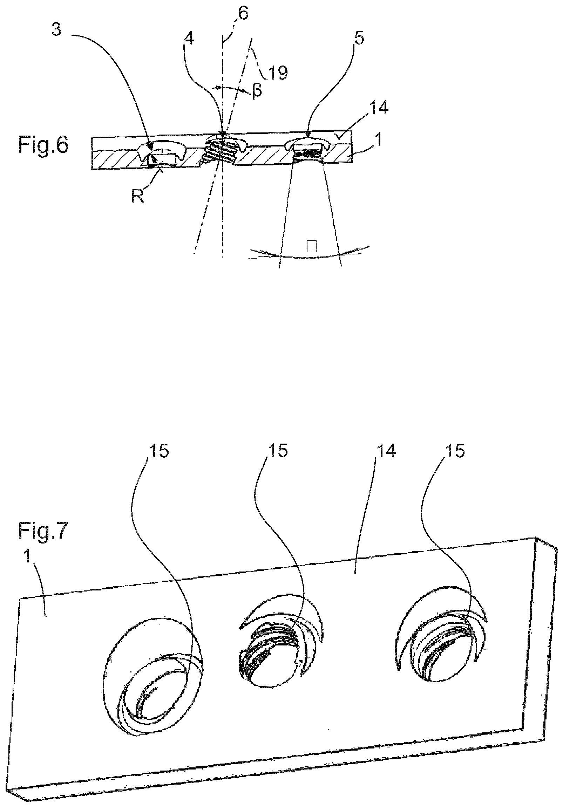

Wie insbesondere aus Figur 6 hervorgeht, sind die Durchgangsöffnungen 3, 4, 5 jeweils mit einem Innengewinde versehen. Die Durchgangsöffnung 3 ist beispielhaft mit einem geraden Gewinde versehen, während die Innengewinde der Durchgangsöffnungen 4 und 5 konisch mit unterschiedlichen Konuswinkeln ausgebildet sind. Insbesondere eine relativ dünne Implantatplatte kann mit einem kurzstreckigen geraden Gewinde versehen sein, während bei einer dickeren Implantatplatte ein langstreckiges konisches Gewinde vorgesehen sein sollte. Wie in der Fig. 6 gezeigt, öffnet sich das konische Innengewinde zu der Seite hin, die mit dem Knochen verbunden wird, bzw. zu der der Kragenflanke 17 gegenüberliegenden oder abgewandten Seite der Implantatplatte 1 hin.

Die Figuren 6 und 7 zeigen des Weiteren, dass die jeweiligen Durchtrittswinkel der Durchgangsöffnungen 3, 4, 5, also die Winkel zwischen der Längsachse 19 der entsprechenden Durchgangsöffnung relativ zur Normalen 6 der Implantatplatte 1, unterschiedlich sind. Während die Durchgangsöffnungen 3 und 5 in Richtung der Normalen eingebracht ist, ist die Durchgangsöffnung 4 in einem Winkel schräg zur Normalen geneigt. Die Innengewinde der Durchgangsöffnungen 3, 4, 5 weisen gegenüber dem Knochengewinde 8 der Knochenschrauben 2a, b ein gewisses Winkelspiel auf, so dass die entsprechende Knochenschraube 2 ohne Spanbildung in einem Winkel schräg zur Längsachse der jeweiligen Durchgangsöffnung eingeschraubt werden kann. Dieses Winkelspiel wird durch die konische Gewindeform der Innengewinde der Durchgangsöffnungen 4 und 5 erzielt. Eine andere zusätzliche oder alternative Möglichkeit zum schrägen Einschrauben wird durch die geneigte Längsachse der Durchgangsöffnungen erzielt.

Nach der Erfindung steht das Knochengewinde 8 sowohl mit dem (in den Figuren nicht dargestellten) Knochen als auch mit dem Innengewinde der jeweiligen Durchgangsöffnung 3, 4, 5 in Einriff. Um einen sicheren Halt der Knochenschraube 2 in dem Innengewinde gewährleisten zu können, ist es von Vorteil, wenn das Knochengewinde 8 möglichst nahe an den Schraubenkopf 9 heranreicht, da dann bei einem vollständigen Eindrehen der Knochenschraube ausreichend Gewindelänge zur Verfügung steht, um mit dem Innengewinde in Eingriff zu stehen. Aus fertigungstechnischen Gründen ist es jedoch schwierig, wenn nicht unmöglich, das Knochengewinde 8 bei der in den Figuren 1, 2, 3 und 4 dargestellten Ausführungsform der Knochenschraube 2 bis nahe an den Schraubenkopf 9 zu führen. Abhilfe schafft hier die in der Figur 5 dargestellte Ausführungsform in Form der Knochenschraube 20. Während bei der in Figur 4 dargestellten Knochenschraube 2 deren Schaft 7 und Kopf 9 einstückig ausgebildet sind, ist die Knochenschraube 20 der Figur 5 zweiteilig aufgebaut. Sie weist den Schaft 7 auf, der endseitig zu einem Teil des Schraubenkopfs 9 aufgeweitet ist. Der schaftseitige Bereich des Schraubenkopfs 9 ist durch einen auf den Schaft 7 aufgepressten Ring 21 gebildet. Dieser weist eine geneigte, zum Schaft 7 gerichtete Fläche 22 auf, die zusammen mit dem Schaft 7 die nutförmige Vertiefung 11 bildet. Die Fläche 22 bildet dabei die äußere Nutflanke aus. Die Knochenschraube 20 wird hergestellt, indem zunächst das Knochengewinde 8 ausgebildet wird und nachfolgend der Ring 21 im Bereich des Schraubenkopfs 9 auf den Schaft aufgepresst wird.

## Patentansprüche

1. Knochenschraube (2, 20) zum winkelvariablen Verschrauben einer Implantatplatte (1) mit einem Knochen, insbesondere einer Implantatplatte (1) nach einem der Ansprüche 7 bis 13, wobei
die Knochenschraube (2, 20) einen Schaft (7) mit einem Knochengewinde (8) und an einem Ende des Schafts (7) einen Schraubenkopf (9) aufweist, und
in dem Schraubenkopf (9) in dessen zum Schaft (7) weisender Unterseite eine in Umfangsrichtung umlaufende nutförmige Vertiefung (11) ausgebildet ist,
**dadurch gekennzeichnet, dass**
die nutförmige Vertiefung (11) eine Nutflanke (12, 22) aufweist, die zum Verklemmen, Verspannen oder Verblocken mit einer Implantatplatte (1) bestimmt ist,
die zum Verklemmen oder Verblocken bestimmte Nutflanke (12, 22) die in radialer Richtung äußere Nutflanke der Vertiefung (11) ist, und
die zum Verklemmen oder Verblocken bestimmte Nutflanke (12, 22) in axialer Richtung gegenüber der Längsachse (13) des Schaftes (7) um einen Winkel α geneigt ist, wobei α in einem Bereich zwischen 30° und 50°, vorzugsweise zwischen 35° und 45° liegt.

2. Knochenschraube (2, 20) zum winkelvariablen Verschrauben einer Implantatplatte (1) mit einem Knochen, insbesondere einer Implantatplatte (1) nach einem der Ansprüche 7 bis 13, wobei
die Knochenschraube (2, 20) einen Schaft (7) mit einem Knochengewinde (8) und an einem Ende des Schafts (7) einen Schraubenkopf (9) aufweist, und
in dem Schraubenkopf (9) in dessen zum Schaft (7) weisender Unterseite eine in Umfangsrichtung umlaufende nutförmige Vertiefung (11) ausgebildet ist,
**dadurch gekennzeichnet, dass**
die nutförmige Vertiefung (11) eine Nutflanke (12, 22) aufweist, die zum Verklemmen, Verspannen oder Verblocken mit einer Implantatplatte (1) bestimmt ist,
die zum Verklemmen oder Verblocken bestimmte Nutflanke (12, 22) die in radialer Richtung äußere Nutflanke der Vertiefung (11) ist, und
die zum Verklemmen oder Verblocken bestimmte Nutflanke (12, 22) kugelsegmentförmig oder konkav mit einem Radius r ausgebildet ist.

3. Knochenschraube (2; 20) nach Anspruch 2, **dadurch gekennzeichnet, dass** der Radius r derart ausgewählt ist, dass der Mittelpunkt der Segmentkrümmung den bestimmungsgemäßen Drehpunkt der Knochenschraube (2; 20) ausbildet, um den diese bei einem schrägen Einschrauben gegenüber einer Normalen einer in der Implantatplatte (1) vorgesehenen Durchgangsöffnung (3, 4, 5) geschwenkt ist.

4. Knochenschraube (2; 20) nach Anspruch 2 oder 3, **dadurch gekennzeichnet, dass** der Radius r so gewählt ist, dass sich der Drehpunkt der Knochenschraube (2; 20) in einem proximalen Schaftabschnitt der Knochenschraube (2; 20) befindet, und zwar in demjenigen Schaftabschnitt, der sich bei einem schrägen Einschrauben in der Implantatplatte (1) befindet, so dass sich der Dreh- oder Schwenkpunkt der Knochenschraube (2; 20) innerhalb der Implantatplatte (1) befindet.

5. Knochenschraube (2; 20) nach einem der vorstehenden Ansprüche, **dadurch gekennzeichnet, dass** der Schraubenkopf (9) zumindest teilweise durch einen am kopfseitigen Ende des Schaftes (7) angeordneten, den Schaft (7) radial umgebenden, insbesondere zum Schaft (7) separaten, Ring (21) ausgebildet ist.

6. Knochenschraube (2; 20) nach Anspruch 5, **dadurch gekennzeichnet, dass** der Ring (21) zumindest die radial äußere Flanke (22) der nutförmigen Vertiefung (11) ausbildet oder dass die nutförmige Vertiefung (11) in dem Ring (21) ausgebildet ist.

7. Implantatplatte (1) zum winkelvariablen Verschrauben einer Knochenschraube (2, 20) mit einem Knochen, insbesondere einer Knochenschraube (2, 20) nach einem der Ansprüche 1 bis 6, wobei
die Implantatplatte (1) wenigstens eine Durchgangsöffnung (3, 4, 5) zur Aufnahme der Knochenschraube (2, 20) aufweist,
die Durchgangsöffnung (3, 4, 5) auf der der zur Anlage am Knochen bestimmten Seite gegenüberliegenden Seite (14) der Implantatplatte (1) von einem in Umfangsrichtung umlaufenden und in axialer Richtung vorstehenden Kragen (15) umgeben ist, und
der Kragen (15) eine Kragenflanke (17) aufweist, die zum Verklemmen, Verspannen oder Verblocken mit der Knochenschraube (2, 20) bestimmt ist,
**dadurch gekennzeichnet, dass**
die zum Verklemmen oder Verblocken bestimmte Kragenflanke (17) die auf der der Durchgangsöffnung (3, 4, 5) abgewandten Seite liegende Kragenflanke (17) ist.

8. Implantatplatte (1) nach Anspruch 7, **dadurch gekennzeichnet, dass** die Kragenflanke (17) in axialer Richtung gegenüber der Längsachse (19) der Durchgangsöffnung (3, 4, 5) um einen Winkel geneigt ist, wobei der Winkel vorzugsweise in einem Bereich zwischen 20° und 40°, bevorzugter zwischen 25° und 35° liegt.

9. Implantatplatte (1) nach Anspruch 7 oder 8, **dadurch gekennzeichnet, dass** die Kragenflanke (17) kugelsegmentförmig oder konvex mit einem Radius R ausgebildet ist.

10. Implantatplatte (1) nach Anspruch 9, **dadurch gekennzeichnet, dass**
der Mittelpunkt der Segmentkrümmung der Kragenflanke (17) auf der Mittelachse der Durchgangsöffnung (3, 4, 5) liegt und /oder der Radius R derart ausgewählt ist, dass der Mittelpunkt der Segmentkrümmung den bestimmungsgemäßen Drehpunkt der Knochenschraube (2; 20) ausbildet, um den diese bei einem schrägen Einschrauben gegenüber einer Normalen der in der Implantatplatte (1) vorgesehenen Durchgangsöffnung (3, 4, 5) geschwenkt ist.

11. Implantatplatte (1) nach einem der vorhergehenden Ansprüche 7 bis 10, **dadurch gekennzeichnet, dass** in der Durchgangsöffnung (3, 4, 5) ein Innengewinde ausgebildet ist.

12. Implantatplatte (1) nach einem der vorhergehenden Ansprüche 7 bis 11, **dadurch gekennzeichnet, dass** in der Durchgangsöffnung (3, 4, 5) ein kegelförmiges Innengewinde ausgebildet ist, mit einem bevorzugten Kegelwinkel γ von 16° oder 18°.

13. Implantatplatte (1) nach einem der vorhergehenden Ansprüche 7 bis 12, **dadurch gekennzeichnet, dass** die Achse (19) wenigstens einer Durchgangsöffnung (3, 4, 5) gegenüber der Normalen (6) der Implantatplatte (1) um einen Winkel β geneigt ist, wobei der Winkel β vorzugsweise in einem Bereich zwischen 0° und 10°, bevorzugter zwischen 2° und 8,2° liegt.

14. System aus wenigstens einer Knochenschraube (2, 20) und einer Implantatplatte (1), wobei
die Knochenschraube (2, 20) zum winkelvariablen Verschrauben der Implantatplatte (1) mit einem Knochen geeignet ist,
die Knochenschraube (2, 20) einen Schaft (7) mit einem Knochengewinde (8) und an einem Ende des Schafts (7) einen Schraubenkopf (9) aufweist,
in dem Schraubenkopf (9) in dessen zum Schaft (7) weisender Unterseite eine in Umfangsrichtung umlaufende nutförmige Vertiefung (11) ausgebildet ist,
die Implantatplatte (1) zum winkelvariablen Verschrauben der Knochenschraube (2, 20) mit dem Knochen geeignet ist,
die Implantatplatte (1) wenigstens eine Durchgangsöffnung (3, 4, 5) zur Aufnahme der Knochenschraube (2, 20) aufweist,
die Durchgangsöffnung (3, 4, 5) auf der der zur Anlage am Knochen bestimmten Seite gegenüberliegenden Seite (14) der Implantatplatte (1) von einem in Umfangsrichtung umlaufenden und in axialer Richtung vorstehenden Kragen (15) umgeben ist, und
der Kragen (15) eine Kragenflanke (17) aufweist, die zum Verklemmen, Verspannen oder Verblocken mit der Knochenschraube (2, 20) bestimmt ist
**dadurch gekennzeichnet, dass**
die nutförmige Vertiefung (11) der Knochenschraube (2, 20) eine Nutflanke (12, 22) aufweist, die zum Verklemmen, Verspannen oder Verblocken mit der Kragenflanke (17) der Implantatplatte (1) bestimmt ist und die zum Verklemmen oder Verblocken bestimmte Kragenflanke (17) die auf der der Durchgangsöffnung (3, 4, 5) abgewandten Seite liegende Kragenflanke (17) ist.

15. System nach Anspruch 14 aus wenigstens einer Knochenschraube (2, 20) nach einem der Ansprüche 1 bis 6 und einer Implantatplatte (1) nach einem der Ansprüche 7 bis 13.

## Claims

1. Bone screw (2, 20) for the angle-variable screwing of an implant plate (1) to a bone, in particular of an implant plate (1) according to one of claims 7 to 13, wherein
the bone screw (2, 20) comprises a shank (7) having a bone thread (8) and a screw head (9) at one end of the shank (7), and
a groove-shaped recess (11) running in the circumferential direction is formed in the screw head (9) in its underside pointing to the shank (7),
**characterized in that**
the groove-shaped recess (11) comprises a groove flank (12, 22) which is provided for clamping, tightening or locking to an implant plate (1),
the groove flank (12, 22) provided for clamping or locking is the outer groove flank of the recess (11) in the radial direction, and
the groove flank (12, 22) provided for clamping or locking is inclined by an angle α in the axial direction with respect to the longitudinal axis (13) of the shank (7), wherein α lies in a range between 30° and 50°, preferably between 35° and 45°.

2. Bone screw (2, 20) for the angle-variable screwing of an implant plate (1) to a bone, in particular of an implant plate (1) according to one of claims 7 to 13, wherein
the bone screw (2, 20) comprises a shank (7) having a bone thread (8) and a screw head (9) at one end of the shank (7), and
a groove-shaped recess (11) running in the circumferential direction is formed in the screw head (9) in its underside pointing to the shank (7),
**characterized in that**
the groove-shaped recess (11) comprises a groove flank (12, 22) which is provided for clamping, tightening or locking to an implant plate (1),
the groove flank (12, 22) provided for clamping or locking is the outer groove flank of the recess (11) in the radial direction, and
the groove flank (12, 22) provided for clamping or locking is configured to be spherical-segment shaped or concave with a radius r.

3. Bone screw (2; 20) according to claim 2, **characterized in that** the radius r is selected such that the midpoint of the segment curvature forms the intended rotation point of the bone screw (2; 20), around which the bone screw (2; 20) is pivoted upon slanted screwing with respect to a normal of a passage opening (3, 4, 5) provided in the implant plate (1).

4. Bone screw (2; 20) according to claim 2 or 3, **characterized in that** the radius r is selected such that the rotation point of the bone screw (2; 20) is located in a proximal shank section of the bone screw (2; 20), that is to say located **in that** section of the shank which is located in the implant plate (1) upon slanted screwing such that the rotation or pivot point of the bone screw (2; 20) is located inside the implant plate (1).

5. Bone screw (2; 20) according to one of the preceding claims, **characterized in that** the screw head (9) is configured at least partially by a ring (21) arranged on the head-side end of the shank (7) and radially surrounding the shank (7), in particular separate to the shank (7).

6. Bone screw (2; 20) according to claim 5, **characterized in that** the ring (21) forms at least the radially outer flank (22) of the groove-shaped recess (11) or that the groove-shaped recess (11) is formed in the ring (21).

7. Implant plate (1) for the angle-variable screwing of a bone screw (2, 20), in particular a bone screw (2, 20) according to any one of claims 1 to 6, to a bone, wherein
the implant plate (1) comprises at least one passage opening (3, 4, 5) for receiving the bone screw (2, 20),
the passage opening (3, 4, 5) on the side (14) of the implant plate (1) lying opposite to the side provided to rest on the bone is surrounded by a collar (15) running in the circumferential direction and protruding in the axial direction, and
the collar (15) comprises a collar flank (17) which is provided for clamping, tightening or locking to the bone screw (2, 20),
the collar flank (17) provided for clamping or locking is the collar flank (17) lying on the side facing away from the passage opening (3, 4, 5).

8. Implant plate (1) according to claim 7, **characterized in that** the collar flank (17) is inclined by an angle in the axial direction with respect to the longitudinal axis (19) of the passage opening (3, 4, 5), wherein the angle lies preferably in a range between 20° and 40°, further preferably between 25° and 35°.

9. Implant plate (1) according to claim 7 or 8, **characterized in that** the collar flank (17) is configured to be spherical-segment shaped or convex with a radius R.

10. Implant plate (1) according to claim 9, **characterized in that** the midpoint of the segment curvature of the collar flank (17) lies on the centre axis of the passage opening (3, 4, 5) and/or the radius R is selected such that the midpoint of the segment curvature forms the intended rotation point of the bone screw (2; 20), around which the bone screw (2; 20) is pivoted upon slanted screwing with respect to a normal of the passage opening (3, 4, 5) provided in the implant plate (1).

11. Implant plate (1) according to one of the preceding claims 7 to 10, **characterized in that** an internal thread is formed in the passage opening (3, 4, 5).

12. Implant plate (1) according to one of the preceding claims 7 to 11, **characterized in that** a conically-shaped internal thread is formed in the passage opening (3, 4, 5), having a preferred taper angle γ of 16° or 18°.

13. Implant plate (1) according to one of the preceding claims 7 to 12, **characterized in that** the axis (19) of at least one passage opening (3, 4, 5) is inclined by an angle β with respect to the normal (6) of the implant plate (1), wherein the angle β lies preferably in a range between 0° and 10°, further preferably between 2° and 8,2°.

14. System of at least one bone screw (2, 20) and an implant plate (1), wherein
the bone screw (2, 20) is suitable for the angle-variable screwing of the implant plate (1) to a bone,
the bone screw (2, 20) comprises a shank (7) having a bone thread (8) and a screw head (9) at one end of the shank (7), and
a groove-shaped recess (11) running in the circumferential direction is formed in the screw head (9) in its underside pointing to the shank (7),
the implant plate (1) is suitable for the angle-variable screwing of the bone screw (2, 20) to the bone, wherein
the implant plate (1) comprises at least one passage opening (3, 4, 5) for receiving the bone screw (2, 20),
the passage opening (3, 4, 5) on the side (14) of the implant plate (1) lying opposite to the side provided to rest on the bone is surrounded by a collar (15) running in the circumferential direction and protruding in the axial direction, and
the collar (15) comprises a collar flank (17) which is provided for clamping, tightening or locking to the bone screw (2, 20),
**characterized in that**
the groove-shaped recess (11) of the bone screw (2; 20) comprises a groove flank (12, 22) which is provided for clamping, tightening or locking to the collar flank (17) of the implant plate (1), and the collar flank (17) provided for clamping or locking is the collar flank (17) lying on the side facing away from the passage opening (3, 4, 5).

15. System according to claim 14 made of at least one bone screw (2, 20) according to one of claims 1 to 6 and an implant plate (1) according to one of claims 7 to 13.

## Revendications

1. Vis pour ostéosynthèse (2, 20) afin de visser avec un angle variable une plaque implantable (1) avec un os, en particulier une plaque implantable (1) selon l'une quelconque des revendications 7 à 13, dans laquelle
la vis pour ostéosynthèse (2, 20) présente une tige (7) avec un filetage d'os (8) et, à une extrémité de la tige (7), une tête de vis (9), et
dans la tête de vis (9), un creux (11) en forme de rainure et faisant tout le tour en direction circonférentielle est réalisé dans le côté inférieur, pointant vers la tige (7), de ladite tête de vis,
**caractérisée en ce que**
le creux en forme de rainure (11) présente un flanc de rainure (12, 22) qui est destiné au coinçage, serrage ou blocage avec une plaque implantable (1),
le flanc de rainure (12, 22) destiné au serrage ou blocage est le flanc de rainure extérieur, en direction radiale, du creux (11), et
le flanc de rainure (12, 22) destiné au serrage ou blocage est incliné d'un angle α en direction axiale par rapport à l'axe longitudinal (13) de la tige (7), α étant compris dans une plage entre 30° et 50°, de préférence entre 35° et 45°.

2. Vis pour ostéosynthèse (2, 20) afin de visser avec un angle variable une plaque implantable (1) avec un os, en particulier une plaque implantable (1) selon l'une quelconque des revendications 7 à 13, dans laquelle
la vis pour ostéosynthèse (2, 20) présente une tige (7) avec un filetage d'os (8) et, à une extrémité de la tige (7), une tête de vis (9), et
dans la tête de vis (9), un creux (11) en forme de rainure et faisant tout le tour en direction circonférentielle est réalisé dans le côté inférieur, pointant vers la tige (7), de ladite tête de vis,
**caractérisée en ce que**
le creux en forme de rainure (11) présente un flanc de rainure (12, 22) qui est destiné au coinçage, serrage ou blocage avec une plaque implantable (1),
le flanc de rainure (12, 22) destiné au serrage ou blocage est le flanc de rainure extérieur, en direction radiale, du creux (11), et
le flanc de rainure (12, 22) destiné au serrage ou blocage est réalisé en forme de segment sphérique ou concave avec un rayon r.

3. Vis pour ostéosynthèse (2 ; 20) selon la revendication 2, **caractérisée en ce que** le rayon r est choisi de telle sorte que le centre de la courbure de segment forme le point de rotation, conforme à sa destination, de la vis pour ostéosynthèse (2 ; 20), point de rotation autour duquel pivote la vis pour ostéosynthèse lors d'un vissage oblique par rapport à une normale à une ouverture de passage (3, 4, 5) prévue dans la plaque implantable (1).

4. Vis pour ostéosynthèse (2 ; 20) selon la revendication 2 ou 3, **caractérisée en ce que** le rayon r est choisi de telle sorte que le point de rotation de la vis pour ostéosynthèse (2 ; 20) se trouve dans un tronçon de tige proximal de la vis pour ostéosynthèse (2 ; 20), et ce dans le tronçon de tige qui se trouve dans la plaque implantable (1) lors d'un vissage oblique de telle sorte que le point de rotation ou de pivotement de la vis pour ostéosynthèse (2 ; 20) se trouve à l'intérieur de la plaque implantable (1).

5. Vis pour ostéosynthèse (2 ; 20) selon l'une quelconque des revendications précédentes, **caractérisée en ce que** la tête de vis (9) est réalisée au moins partiellement par un anneau (21) agencé au niveau de l'extrémité côté tête de la tige (7), entourant de façon radiale la tige (7), en particulier séparé de la tige (7).

6. Vis pour ostéosynthèse (2 ; 20) selon la revendication 5, **caractérisée en ce que** l'anneau (21) forme au moins le flanc de façon radiale extérieur (22) du creux en forme de rainure (11) ou **en ce que** le creux en forme de rainure (11) est réalisé dans l'anneau (21).

7. Plaque implantable (1) destinée à visser avec un angle variable une vis pour ostéosynthèse (2, 20) avec un os, en particulier une vis pour ostéosynthèse (2, 20) selon l'une quelconque des revendications 1 à 6, laquelle plaque implantable (1) présente au moins une ouverture de passage (3, 4, 5) destinée à recevoir la vis pour ostéosynthèse (2, 20), laquelle ouverture de passage (3, 4, 5) est entourée sur le côté (14), à l'opposé du côté destiné à l'appui au niveau de l'os, de la plaque implantable (1) par un collet (15) faisant le tour en direction circonférentielle et dépassant en direction axiale et lequel collet (15) présente un flanc de collet (17) qui est destiné au coinçage, serrage ou blocage avec la vis pour ostéosynthèse (2 ; 20),
**caractérisée en ce que** le flanc de collet (17) destiné au coinçage, serrage ou blocage est le flanc de collet (17) situé sur le côté éloigné de l'ouverture de passage (3, 4, 5).

8. Plaque implantable (1) selon la revendication 7, **caractérisée en ce que** le flanc de collet (17) est incliné d'un certain angle en direction axiale par rapport à l'axe longitudinal (19) de l'ouverture de passage (3, 4, 5), lequel angle est compris de préférence dans une plage entre 20° et 40°, de préférence entre 25° et 35°.

9. Plaque implantable (1) selon la revendication 7 ou 8, **caractérisée en ce que** le flanc de collet (17) est réalisé en forme de segment sphérique ou convexe avec un rayon R.

10. Plaque implantable (1) selon la revendication 9, **caractérisée en ce que** le centre de la courbure de segment du flanc de collet (17) est situé sur l'axe médian de l'ouverture de passage (3, 4, 5) et/ou le rayon R est choisi de telle sorte que le centre de la courbure de segment forme le point de rotation, conforme à sa destination, de la vis pour ostéosynthèse (2 ; 20), point de rotation autour duquel pivote celle-ci lors d'un vissage oblique par rapport à une normale à une ouverture de passage (3, 4, 5) prévue dans la plaque implantable (1).

11. Plaque implantable (1) selon l'une quelconque des revendications précédentes 7 à 10, **caractérisée en ce qu'**un filetage intérieur est réalisé dans l'ouverture de passage (3, 4, 5).

12. Plaque implantable (1) selon l'une quelconque des revendications précédentes 7 à 11, **caractérisée en ce qu'**un filetage intérieur conique, avec un angle de cône γ préféré de 16° ou 18°, est réalisé dans l'ouverture de passage (3, 4, 5).

13. Plaque implantable (1) selon l'une quelconque des revendications précédentes 7 à 12, **caractérisée en ce que** l'axe (19) d'au moins une ouverture de passage (3, 4, 5) est incliné d'un angle β par rapport à la normale (6) à la plaque implantable (1), lequel angle β se trouve de préférence dans une plage entre 0° et 10°, de préférence entre 2° et 8,2°.

14. Système constitué d'au moins une vis pour ostéosynthèse (2, 20) et d'une plaque implantable (1), dans lequel
la vis pour ostéosynthèse (2, 20) est appropriée pour visser avec un angle variable la plaque implantable (1) avec un os,
la vis pour ostéosynthèse (2, 20) présente une tige (7) avec un filetage d'os (8) et, à une extrémité de la tige (7), une tête de vis (9),
dans la tête de vis (9), un creux (11) en forme de rainure et faisant tout le tour en direction circonférentielle est réalisé dans le côté inférieur, pointant vers la tige (7), de ladite tête de vis,
la plaque implantable (1) est appropriée pour visser avec un angle variable la vis pour ostéosynthèse (2, 20) avec l'os,
la plaque implantable (1) présente au moins une ouverture de passage (3, 4, 5) destinée à recevoir la vis pour ostéosynthèse (2, 20),
l'ouverture de passage (3, 4, 5) est entourée sur le côté (14), à l'opposé du côté destiné à l'appui au niveau de l'os, de la plaque implantable (1) par un collet (15) faisant le tour en direction circonférentielle et dépassant en direction axiale et
le collet (15) présente un flanc de collet (17) qui est destiné au coinçage, serrage ou blocage avec la vis pour ostéosynthèse (2, 20),
**caractérisé en ce que** le creux en forme de rainure (11) de la vis pour ostéosynthèse (2, 20) présente un flanc de rainure (12, 22) qui est destiné au coinçage, serrage ou blocage avec le flanc de collet (17) de la plaque implantable (1) et le flanc de collet (17) destiné au coinçage, serrage ou blocage est le flanc de collet (17) situé sur le côté éloigné de l'ouverture de passage (3, 4, 5).

15. Système selon la revendication 14 constitué d'au moins une vis pour ostéosynthèse (2, 20) selon l'une des revendications 1 à 6 et d'une plaque implantable (1) selon l'une des revendications 7 à 13.
